# EUROPEAN PATENT SPECIFICATION

(11) **EP 0 384 562 B1**
(45) Date of publication and mention of the grant of the patent: **17.07.1996**
(21) Application number: 90300337.4
(22) Date of filing: 11.01.1990
(51) Int. Cl.: A61B 17/14

(54) **Tibial surface shaping guide for knee implants**
Lehre zur Formung der Tibiaoberfläche für Kniegelenks-Endoprothese
Guide pour façonner la surface du tibia pour recevoir une prothèse de genou

(30) Priority: 30.01.1989 US 303709
(43) Date of publication of application: 29.08.1990
(73) Proprietor: Wright Medical Technology, Inc., Arlington, Tennessee (US)
(72) Inventor: Whiteside, Leo Allen, Bridgeton, Missouri (US); Stamp, Carl Martin, Cordova, Tennessee (US)
(74) Representative: Dowden, Marina

(56) References cited:
- EP-A- 0 121 142
- EP-A- 0 194 014
- US-A- 4 487 203
- US-A- 4 703 751
- US-A- 4 736 737

## Description

This invention relates to an apparatus for shaping the proximal surface of a human tibia, which enables resection to be accomplished without sacrifice of the anterior cruciate ligament.

In replacing a knee joint which has been damaged due to disease or trauma, it is very important that the prosthesis used to replace the damaged portion of the joint be properly aligned with respect to the bone to which the prosthesis is fixed. Particular problems are encountered when resection of the proximal surface of the tibia is required, in that most shaping instrumentation requires sacrifice of the anterior cruciate whereas in most replacements the stability of the knee with replacement implants in place would be significantly improved by retaining the ligament.

US-A-4,487,203 in the name of Gary W. Androphy discloses a triplanar knee resection system which includes a single guide member for use in making the bone resections which includes an alignment opening for co-operating with a guide rod, the guide rod having a 90° angle bend and being adapted to be inserted into the femur for use in aligning the guide member. In one aspect, the guide rod is designed in such manner that it remains inserted in the femur during the surgical procedure, thereby establishing a common reference point for the bone resection. The guide member co-operates with the guide rod to enable resection of the knee joint with the knee always in flexion. A second identical guide rod is provided along with a tibia adaptor and a tibia bar for use as a guide member in resecting the proximal tibia. A femur bar is provided for use of the guide member and femur guide rod in making the resection of the distal femur. In a second aspect, the femur guide bar is designed to compensate for the appropriate valgus angle of the femur relative to an imaginary axis extending through the hip joint, knee joint and ankle joint. In another aspect, the triplanar knee system achieved equal flexion and extension gaps at the resected knee joint. Put in another aspect, the system ensures proper alignment of the distal femural resection and approximal tibular resection, although these two resections are independently aligned. In a method aspect, a common reference is established for resecting the anteria and posteria distal femural condyles and the distal femur. This common reference is used to provide equal flexion and extension gaps. In another aspect of the method, the alignment of the guide member of resecting the distal femur is accomplished without the use of any "eyeball" techniques.

More particularly, the system of the latter US Patent to Gary W. Androphy provides a tibial surface shaping apparatus having a tibia guide rod with an off-set handle, a cutting guide body, means for adjustably fixing the cutting guide body to the handle and a tibia plateau surface shaping guide carried by said guide rod. It is to be noted that the off-set handle portion is in fact perpendicular and that the adjustable guide is not adjustable with respect to the longitudinal axis of the rod portion.

To enable a surgeon to shape the proximal tibia to receive a tibial component of a total knee joint prosthesis, Leo A. Whiteside, one of the named inventors herein, developed a method and apparatus for shaping a proximal tibial surface which is claimed in U.S. Patent No. 4,467,801. That patent teaches the use of an intramedullary alignment guide which provides the surgeon with a means for determining the central long axis of the tibia and a means by which the surgeon can shape the proximal surface of the tibia relative to that axis by attaching a planer or similar shaping instrument to that alignment guide. The anterior cruciate ligament is generally sacrificed using that method and apparatus.

We have sought to provide a method and apparatus for shaping the peripheral surface of a human tibia which overcome the problems heretofore experienced in connection with resection of the tibia for implantation of a prosthesis while sparing the anterior cruciate ligament, and which provide for simple but accurate alignment.

According to our invention, we provide a tibial surface shaping apparatus comprising an alignment rod portion (12) with an offset handle (14) together forming an intramedullary alignment guide (10), a cutting guide body (70), means (72, 26) for adjustably fixing the cutting guide body (70) to the handle (14), and a tibia plateau surface shaping guide (74) carried by said guide body (70), characterised in that:
(A) the intramedullary alignment guide ( 10 ) consists of:
   (1) the alignment rod portion (12) adapted to be positioned in a hole reamed in the tibial intrameduallary canal, said hole commencing at a point anterior to the anterior cruciate ligament and running adjacent and parallel to the anterior cortex of the intramedullary canal, and
   (2) the offset handle portion (14), wherein said handle portion (14) is of noncircular cross section integral with said alignment rod portion (12), the angle between said alignment rod portion (12) and said offset handle portion (14) is between 9° and 12° and is such that said handle portion (14) is perpendicular to the plane of the tibia plateau when the alignment rod portion (12) is in place,
(B) the cutting guide body (70) has an opening (71) adapted to fit over said offset handle portion (14) of said intramedullary alignment guide (10),
(C) the means (72,26) for adjustably fixing said cutting guide body (70) to the handle (14) is adjustable so as to effect proper alignment with respect to the central long axis of the tibia and the alignment rod portion (12) of said intramedullary alignment guide (10),
(D) at least one tibial plateau surface shaping guide (74) carried by said cutting guide body (70) has a cutting guide slot (75) thereon to permit at least one shaping step of the proximal tibia surface, and said cutting guide body (70) remains adjustably fixed to the offset handle portion (14) of the intramedullary alignment guide (10) during the shaping process.

According to one aspect of the present invention, the tibial plateau surface shaping guide carried on the cutting guide body allows shaping to be carried out relative to the intramedullary alignment guide. According to another aspect, the present invention provides an improved means for alignment of the tibial surface shaping apparatus vertically and in the anterior/posterior direction, providing a surgeon with a tibial plateau surface of the cutting guide body connected to the tibial surface shaping apparatus so that it is attached and aligned easily during the surgical procedure.

The present invention further provides a tibial surface shaping apparatus wherein the shaping guide is vertically adjustable and which provides for alignment to permit accurate shaping of the tibial plateau. Such alignment coupled with attachment to an alignment rod portion located along the anterior cortex of the intramedullary canal of the tibia provides points of reference for all shaping operations.

The cutting guide body of the present invention is adjustable with respect to the offset handle portion of the intramedullary alignment guide so that the amount of resection of the tibial plateau can be controlled readily by the surgeon by raising or lowering of the cutting guide body. The cutting guide body which attaches to the offset handle portion of the intramedullary alignment guide, remains fixed to the offset handle portion after the cutting guide body is adjusted. The cutting guide body contains at least one shaping guide for resection of the proximinal surface of the tibia which is adjustably secured to the cutting guide body.

Briefly summarized, the invention contemplates the use of an intramedullary alignment guide wherein the alignment rod portion is inserted into the intramedullary canal of the tibia from a point anterior to the anterior cruciate ligament and extending along the anterior cortex of the intramedullary canal of the tibia, preferably through the isthmus of the canal and a short distance beyond the isthmus into the widening part of the canal below the isthmus. The alignment rod portion generally has a length of about 25.4 cm (10 inches) for this purpose.

The above and other objects, features and advantages of the present invention will become apparent to those skilled in the art upon an examination of the following description and drawings which are illustrative of the present invention.
In the Drawings:
FIG. 1 is a side view of an intramedullary alignment guide of the present invention.
FIG. 2 is a crossectional view along Line 2-2 of FIG. 1,
FIG. 3 is a side perspective view showing an intramedullary alignment guide with the alignment rod portion inserted into the intramedullary canal of a tibia (with parts broken away), and
FIG. 4 is a side perspective view showing a tibial surface shaping apparatus of the invention comprising a cutting guide body attached to an intramedullary alignment guide inserted along the central axis of a human tibia.

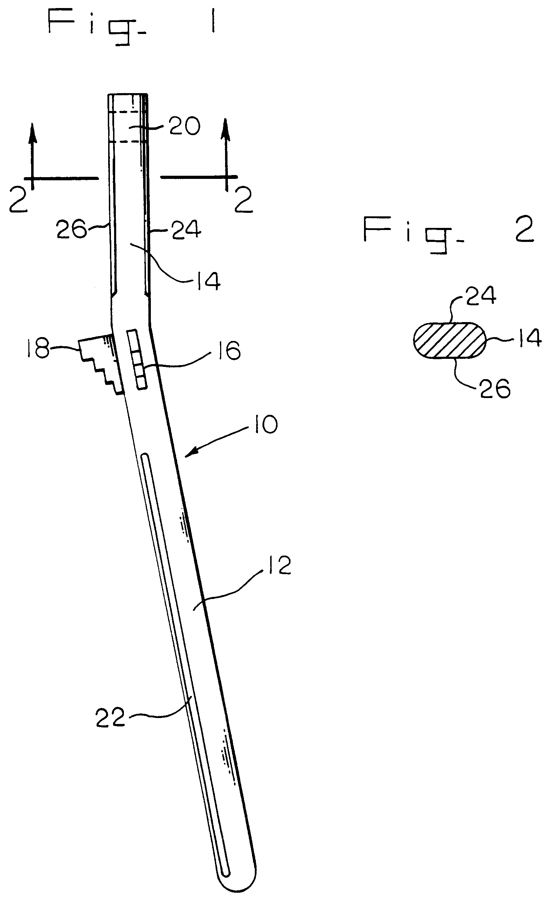

Referring to the drawings and specifically to Figure 1, there is seen an intramedullary alignment guide 10 having an alignment rod portion 12 adapted to be positioned in the intramedullary canal of a tibia. The intramedullary alignment guide 10 has an offset handle portion 14 attached to and set at a pre-selected angle with respect to the axis of the alignment rod portion 12. Locking fins 16 and 18 are provided for anchoring the intramedullary alignment guide 10 at a selected orientation within the intramedullary canal. Generally a third locking fin would be positioned on the side of the alignment rod portion 12 opposite of fin 16.

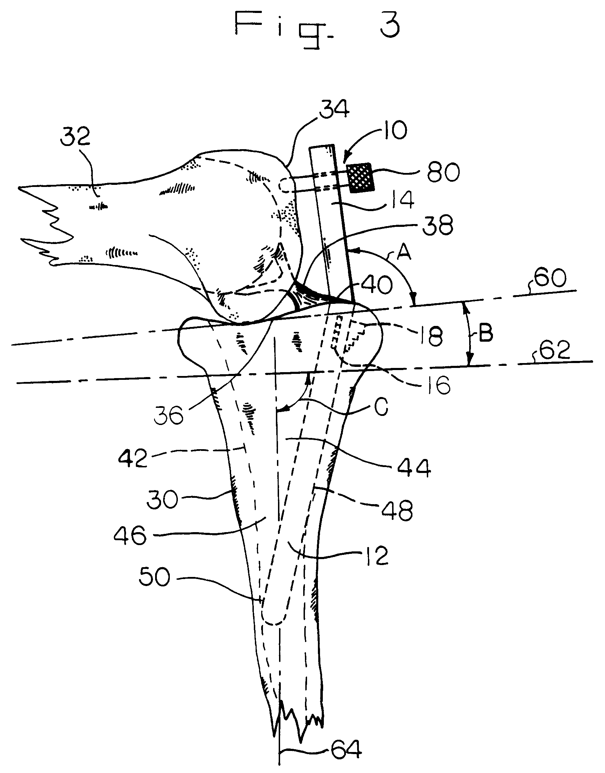

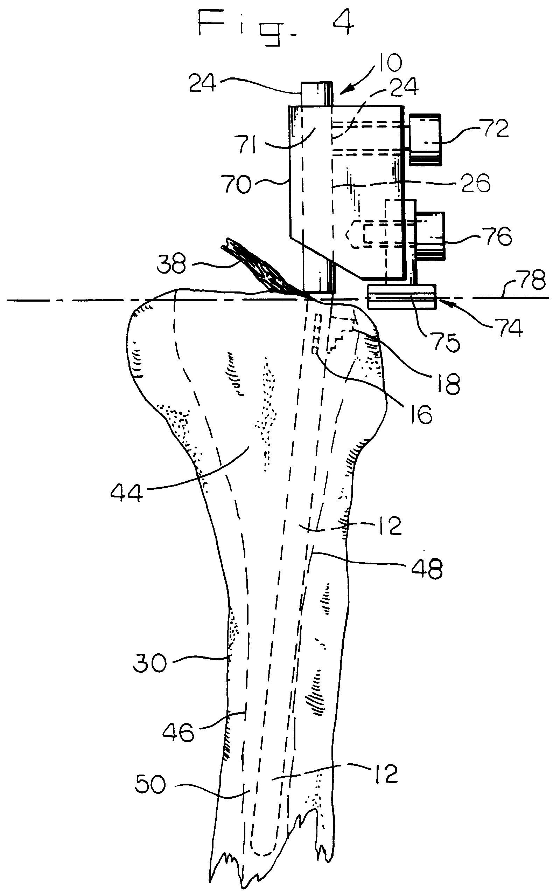

A circular bore 20 is provided through the offset handle portion 14 to assist the surgeon in rotating the intramedullary alignment guide during insertion, if necessary, and for removing the alignment rod portion from the tibia after use. Optional flutes or indentations may be provided on the stem of the alignment rod portion as indicated at 22. Flattened portions 24 and 26 on the offset handle portion 14 are provided in order to allow placement on the offset handle portion of a cutting guide body 70 which is thereby prevented from rotation on the offset handle. The flattened sides may be oriented as desired. It will also be apparent to one skilled in the art that rotation could be prevented by one flattened surface or a combination of grooves or keys. In Figure 3, there is shown a knee joint having a femur 32 which has a distal condylar surface 34 and a tibia 30 which has a proximal tibial plateau 36. Anterior cruciate ligament 38 joins tibia 30 to femur 32. The tibia comprises exterior hard cortical bone 42 and interior soft cancellous bone in the intramedullary canal 44. The posterior cortex joining the cortical and cancellous bone is indicated by numeral 46 and there is a similar cortex 48 along the anterior surface of the intramedullary canal.

As also seen in Figure 3, alignment rod portion 12 extends through an opening 40 which is made anterior to the anterior cruciate ligament 38 and extends down the intramedullary canal along cortex 48 somewhat beyond isthmus 50 which is the narrowest part of the intramedullary canal. Locking fins 16 and 18 serve to prevent rotation and to stabilize the alignment of the tibial surface shaping apparatus.

It is desirable that the offset handle portion 14 of intramedullary alignment guide 10 be designed so that angle A between the axis of the offset handle portion 14 and line 60, which represents the plane of the tibial plateau surface, is 90 degrees. Angle B indicates the degree of tilt between tibial plateau 36 and line 62 which is perpendicular to axis 64 of the tibia as indicated by angle C. In order for the surgeon to obtain proper alignment, it is necessary to provide him with a series of intramedullary alignment guides 10 having an angle between the axis of the alignment rod portion 12 and offset handle portion 14 which varies between about 9 degrees and 12 degrees to take into account individual differences in the shape of the tibia. The proper angle is generally determined by the surgeon using X-rays during the course of pre-operative planning.

As shown in Figure 4, the tibial surface shaping apparatus of the present invention comprises a cutting guide body 70 which contains an opening 71 adapted to slidably fit over the offset handle portion 14 and which is adjustably secured in place by means of a threaded set screw 72 which engages a flattened portion 26 of the offset handle portion 14. Other adjustable securing means can be substituted by those skilled in the art. Carried by cutting guide body 70 is a shaping guide 74 which contains a cutting guide slot 75 for guiding a resection tool. A shaping guide 74 is adjustably secured to cutting guide body 70, preferably by a threaded set screw 76. Shaping guide 74 may thus be raised or lowered to position gutting guide slot 75 along the plane of resection indicated by line 78.

The manner in which the method of shaping the proximal surface of a human tibia may be carried out will now be described. The pre-operative procedures for radiographically determining the central long axis of the tibia and the angle at which resection of the proximal tibia plateau surface is to be made with respect to that axis is the same as is typically employed in other methods known to surgeons skilled in the art. As noted above, the angle B of inclination between the tibial plateau and a line perpendicular to the axis of the tibia is determined. The entry point 40 for the intramedullary reamer is identified. Operatively, the usual surgical approach is made after the interior aspect of the knee is exposed. The knee is flexed, generally to 100 degrees, so that the surfaces of the femur and tibia can be visualized. The preferred intramedullary alignment guide with an alignment rod portion with a diameter of 9.4 mm (0.370 inches) and a suitable drill bit is selected by the surgeon to accommodate such an alignment diameter. The drill is advanced along the cortical surface 48 thereby removing soft cancellous bone from the intramedullary canal. A reamer may be used in forming the opening for entry of the alignment rod portion 12. The opening should preferably be extended through the intramedullary canal to the isthmus of the tibia.

Following preparation of the tibia for the insertion of the intramedullary alignment guide 10, the alignment rod portion 12 is inserted into the tibia to a location where the fins almost touch the proximal surface. A small diameter pin 80 is then inserted into circular bone 20 on the intramedullary alignment guide 10. Using small diameter pin 80 as a guide, rotational alignment is set by aligning the pin 80 with the intracondylar notch of femur 32. The intramedullary alignment guide is then driven into place, with locking fins 16 and 18 engaging the proximal surface of the tibia.

The cutting guide body, intramedullary alignment guide, shaping guide and associated components are all preferably manufactured from a suitable surgical grade of stainless steel or other metal commonly employed by those skilled in the art to construct surgical tools for use in contact with the body. The exact composition of the materials of construction do not form part of the present invention as long as it performs the desired function.

The manner in which the tibial surface shaping apparatus of the present invention may be used will now be described. The present invention relies on the use of an intramedullary alignment guide. The intramedullary alignment guide 10 is inserted into the opening for entry of the alignment rod portion in tibia 30 up to the point where fins almost tough the proximal surface of tibia 30. The rotational alignment of offset handle portion 14 is adjusted and alignment rod portion 12 is then driven into the tibia using a mallet until the locking fins 16 and 18 are embedded in the cortical bone of the tibia. The cutting guide body 70 is then secured in the desired position on the offset handle portion 14. The depth of the resection is then adjusted by raising or lowering shaping guide 74 and tightening threaded set screw 76.

Thereafter the surgeon is able to shape the tibial plateau in accordance with conventional surgical procedures using the cutting guide slots 75. As will be appreciated, severance of the anterior cruciate ligament 38 and the bony island to which it is attached is not required using the apparatus and method of this invention.

After aligning cutting guide body 70 relative to the tibial plateau, a conventional shaping means, such as an oscillating saw or a hand saw (not shown), is then introduced into cutting guide slot 75 to enable the surgeon to resect the proximal tibial plateau accurately along the plane indicated by line 78. The cuts are designed to fit the particular prosthesis to be fixed to the proximal tibial surface. The prosthesis is affixed to the tibia in conventional fashion using appropriate pins, posts or fins, which may be integral with the prosthesis, depending on the particular design. The surgeon can cut such holes or slots as are deemed desirable for securing a particular prosthesis. Appropriate slots or holes can be provided in a suitable cutting guide body also attachable to offset handle portion 14 to assist in such cutting procedures. When shaping is completed, the intramedullary alignment guide is removed and the prosthesis installed in accordance with conventional techniques.

## Claims

1. A tibial surface shaping apparatus comprising an alignment rod portion (12) with an offset handle (14) together forming an intramedullary alignment guide (10), a cutting guide body (70), means (72,26) for adjustably fixing the cutting guide body (70) to the handle (14), and a tibia plateau surface shaping guide (74) carried by said guide body (70), characterised in that:
(A) the intramedullary alignment guide (10) consists of:
(1) the alignment rod portion (12) adapted to be positioned in a hole reamed in the tibial intramedullary canal, said hole commencing at a point anterior to the anterior cruciate ligament and running adjacent and parallel to the anterior cortex of the intramedullary canal, and
(2) the offset handle portion (14), wherein said handle portion (14) is of noncircular cross section integral with said alignment rod portion (12), the angle between said alignment rod portion (12) and said offset handle portion (14) is between 9° and 12° and is such that said handle portion (14) is perpendicular to the plane of the tibia plateau when the alignment rod portion (12) is in place,
(B) the cutting guide body (70) has an opening (71) adapted to fit over said offset handle portion (14) of said intramedullary alignment guide (10),
(c) the means (72,26) for adjustably fixing said cutting guide body (70) to the handle (14) is adjustable so as to effect proper alignment with respect to the central long axis of the tibia and the alignment rod portion (12) of said intramedullary alignment guide (10),
(D) at least one tibial plateau surface shaping guide (74) carried by said cutting guide body (70) has a cutting guide slot (75) thereon to permit at least one shaping step of the proximal tibia surface, and said cutting guide body (70) remains adjustably fixed to the offset handle portion (14) of the intramedullary alignment guide (10) during the shaping process.

2. The tibial surface shaping apparatus as claimed in claim 1, wherein the shaping guide is adjustable vertically relative to said cutting guide body.

3. The tibial surface shaping apparatus as claimed in claim 2, wherein said alignment rod portion of the intramedullary alignment guide is of such dimension so as to be adapted to extend through the isthmus of said intramedullary canal.

4. The tibial shaping apparatus as claimed in claim 3, wherein the alignment rod portion has a length of approximately 25.4 mm and a diameter of approximately 9.4 mm.

## Patentansprüche

1. Vorrichtung zum Formen der Tibiaoberfläche mit einem Ausrichtstangenabschnitt (12) mit einer abgebogenen Handhabe (14), welche zusammen eine intramedulläre Ausrichtführung (10) bilden, einem Schnittführungskörper (70), Mitteln (72, 26) zum verstellbaren Befestigen des Schnittführungskörpers (70) an der Handhabe (14) und einer von dem Führungskörper (70) getragenen Formführung (74) für die Tibiaplateauoberfläche,
**dadurch gekennzeichnet,**
(A) daß die intramedulläre Ausrichtführung (10) aus
(1) dem Ausrichtstangenabschnitt (12), der dafür ausgebildet ist, in einer aufgeweiteten Öffnung des tibialen Knochenmarkkanals positioniert zu werden, wobei die Öffnung an einem Punkt vor dem vorderen Kreuzband beginnt und entlang und parallel zu dem vorderen Cortex des Knochenmarkkanals verläuft, und
(2) dem abgebogenen Handhabungsabschnitt (14) besteht, der einen nicht-kreisförmigen Querschnitt aufweist und integral mit dem Ausrichtstangenabschnitt (12) verbunden ist, wobei der Winkel zwischen dem Ausrichtstangenabschnitt (12) und dem abgebogenen Handhabungsabschnitt (14) zwischen 9° und 12° liegt und so bemessen ist, daß der Handhabungsabschnitt (14) senkrecht zur Ebene des Tibiaplateaus steht, wenn sich der Ausrichtstangenabschnitt (12) an seinem Platz befindet,
(B) daß der Schnittführungskörper (70) eine Öffnung (71) besitzt, die derart ausgebildet ist, daß sie über den abgebogenen Handhabungsabschnitt (14) der intramedullären Ausrichtführung (10) paßt,
(C) daß die Mittel (72, 26) zum verstellbaren Befestigen des Schnittführungskörpers (70) an der Handhabe (14) derart verstellbar sind, daß mit ihnen eine korrekte Ausrichtung bezüglich der Mittellängsachse der Tibia und des Ausrichtstangenabschnitts (12) der intramedullären Ausrichtführung (10) bewirkbar ist, und
(D) daß wenigstens eine von dem Schnittführungskörper (70) getragene Formführung (74) für die Tibiaplateauoberfläche einen Schnittführungsschlitz (75) aufweist, um wenigstens einen Formungsschritt der proximalen Tibiaoberfläche zu erlauben, wobei der Schnittführungskörper (7) während des Formungsprozesses verstellbar an dem abgebogenen Handhabungsabschnitt (14) der intramedullären Ausrichtführung (10) befestigt bleibt.

2. Vorrichtung zum Formen der Tibiaoberfläche nach Anspruch 1, dadurch gekennzeichnet, daß die Formführung relativ zu dem Schnittführungskörper vertikal verstellbar ist.

3. Vorrichtung zum Formen der Tibiaoberfläche nach Anspruch 2, dadurch gekennzeichnet, daß der Ausrichtstangenabschnitt der intramedullären Ausrichtführung solche Abmessungen aufweist, daß er sich durch den Isthmus des Knochenmarkkanals hindurch zu erstrecken vermag.

4. Vorrichtung zum Formen der Tibiaoberfläche nach Anspruch 3, dadurch gekennzeichnet, daß der Ausrichtstangenabschnitt eine Länge von ungefähr 25,4 mm und einen Durchmesser von ungefähr 9,4 mm besitzt.

## Revendications

1. Appareil de façonnage de la surface du tibia comprenant une partie (12) à tige d'alignement munie d'une poignée (14) décalée formant ensemble un guide d'alignement intramédullaire (10), un corps (70) de guide tranchant, des moyens (72, 26) pour fixer de façon réglable le corps (70) de guide tranchant à la poignée (14), et un guide (74) de façonnage de surface de plateau tibial porté par ledit corps (70) de guide, caractérisé en ce que :
(A) le guide (10) d'alignement intramédullaire est constitué :
(1) de la partie à tige d'alignement (12) adaptée à être positionnée dans un trou alésé dans le canal intramédullaire tibial, ledit trou commençant en un point antérieur au ligament croisé antérieur et d'étendant de façon adjacente et parallèle au cortex antérieur du canal intramédullaire, et
(2) de la partie (14) à poignée décalée, ladite partie (14) à poignée présentant une section transversale non circulaire solidaire de ladite partie (12) à tige d'alignement, l'angle entre ladite partie (12) à tige d'alignement et ladite partie (14) à poignée décalée étant compris entre 9° et 12° et étant tel que ladite partie (14) à poignée est perpendiculaire au plan du plateau tibial lorsque la partie (12) à tige d'alignement est en place,
(B) le corps (70) de guide tranchant a une ouverture (71) adaptée à s'ajuster sur ladite partie (14) à poignée décalée dudit guide (10) d'alignement intramédullaire,
(C) les moyens (72, 26) permettant de fixer de façon réglable ledit corps (70) de guide tranchant sur la poignée (14) sont réglables de façon à permettre un alignement approprié par rapport a l'axe longitudinal central du tibia et à la partie (12) à tige d'alignement dudit guide (10) d'alignement intramédullaire,
(D) au moins un guide (74) de façonnage de la surface du plateau tibial porté par ledit corps (70) de guide tranchant présente une rente (75) pour guide tranchant disposée sur celui-ci pour permettre au moins une étape de façonnage de la surface proximale du tibia, et ledit corps (70) de guide tranchant reste fixé de façon réglable à la partie (14) à poignée décalée dudit guide (10) d'alignement intramédullaire pendant le processus de façonnage.

2. Appareil de façonnage de la surface du tibia selon la revendication 1, dans lequel le guide de façonnage est réglable verticalement par rapport audit corps de guide tranchant.

3. Appareil de façonnage de la surface du tibia selon la revendication 2, dans lequel ladite partie à tige d'alignement du guide d'alignement intramédullaire a une dimension telle qu'elle est adaptée à s'étendre à travers l'isthme dudit canal intramédullaire.

4. Appareil de façonnage du tibia selon la revendication 3, dans lequel la partie à tige d'alignement a une longueur d'environ 25,4 mm et un diamètre d'environ 9,4 mm.
